# EUROPEAN PATENT APPLICATION

(11) **EP 3 492 092 A1**
(43) Date of publication of application: **05.06.2019**
(21) Application number: 17836448.5
(22) Date of filing: 26.07.2017
(51) Int. Cl.: A61K 36/41, A61K 31/63, A61P 17/00, A61P 31/10

(54) **METHOD FOR PREPARING A PRODUCT BASED ON LEAVES OF THE SEDUM TELEPHIUM PLANT FOR THE TREATMENT OF NAILS INFECTED WITH ONYCHOMYCOSIS**

(30) Priority: 01.08.2016 ES 201600663
(71) Applicant: González Hernández, Ángel, 29003 Málaga (ES)
(72) Inventor: FERNÁNDEZ LEÓN, Marta, 29003 Málaga (ES); GONZÁLEZ HERNÁNDEZ, Ángel, 29003 Málaga (ES)
(74) Representative: Garavelli, Paolo
(86) International application number: PCT/ES2017/000095
(87) International publication number: WO 2018/024923

(57) **Abstract**

Procedure for preparation of a product based on the leaves of the plant Sedum telephium for the treatment of nails infected with onychomycosis.

The procedure consists in strip away the skin of the leaf of the Sedum telephium plant of one of its faces, after which the stripped face is impregnated with sulfanilamide powder and then applied as a dressing on nails infected with onychomycosis.

It is also used for topical application in the form of cream or ointment on nails infected with onychomycosis.

## Description

### SECTOR OF THE TECHNIQUE

The present invention relates to the preparation of a product by which infection of the human nail known as onychomycosis is cured.

The main object of this invention is the use of the plant leaf Sedum telephium which together with sulfanilamide powder impregnated in adequate proportion achieves the cure of onychomycosis.

### BACKGROUND OF THE INVENTION

Plaster for the treatment of onychomycosis is already known. For example, WO99 / 40955 A discloses a pressure sensitive steel matrix patch for the treatment of onychomycosis. This device for treating fungal infections of the nails is comprised of an occlusive backing layer and a pressure sensitive adhesive matrix layer, wherein an effective amount of an antifungal agent is evenly dispersed, optionally with a chemical improver.

Another treatment for treating onychomycosis is described in US5464610 A. Such a treatment uses a plaster preparation comprising salicylic acid or a salt, ester or mixture thereof. The plaster preparation is connected to and conveys the salicylic acid which is present in the plaster preparation in an amount in the range of 10 to 80% by weight of the preparation.

Compositions and treatments for fingernail eviction and treating nail infections are described in US5993790 A, wherein a topical nail enamel composition comprising water based nail polish, a preservative, urea and a natural additive. This nail polish composition is suitable for treatment of fungal, yeast and bacterial infections of nails and nail matrices.

US 5753256 A discloses a plaster for the treatment of nail fungus, comprising a flexible cover film, a layer of an acrylate polymer matrix, inseparably bound to the cover film and comprising an active compound selected from miconazole, econazole, isoconazole, thioconazole, terconazole, oxiconazole, ketoconazole, itraconazole, tolcyclate, sulbentine, haloprogine, griseofulvin, cyclopirox, terbinafine, and salts of these compounds.

US5181914 A discloses an adhesive gel pad for treating onychomycosis comprising an occlusive layer, a permeation enhancer and an antifungal agent. US6303140 A discloses a poultice for treating warts, eyes of chickens and calluses containing salicylic acid as the active ingredient. US6303140 A also discloses that when the plaster is used to treat onmyicosis antifungals such as clotrimazole, butenafine, terbinafine or miconazole may be added.

On the other hand, patents describing the use of plants as curative agents are well known, among them mention may be made of the Spanish patent ES2104516 of Emeterio Perez Ruiz in which the use of a plant of the same family and genus is patented (Crasulacea, Sedum) of which is claimed in this patent. In it refers to the use of the juice of the leaves of the plant Sedum album L mixed together with other ingredients and claiming it as an anti-inflammatory lotion.

### CURRENT TREATMENT OF ONYCHOMYCOSIS

In a publication of the National Health System. Volume 32, No. 3/2008, Drs Larruskain, Idígoras and others, say the following about onychomycosis: Nail infection caused by fungi or onychomycosis (OM) is a very frequent process. Numerous studies from developed countries give prevalence figures between 2-18% of the population. Onychomycosis are infections of the nails produced by three types of fungi: dermatophytes, yeasts and non-dermatophytic molds. The microbiological diagnosis is essential for its treatment, since this varies according to the etiologic agent and the type of nail injury. In onychomycosis caused by dermatophytes, the most frequent and usually affecting the toenails, topical treatment is usually not enough, most occasions requiring prolonged oral treatment. The combination of oral terbinafine and amorolfine in lacquer is the therapeutic regimen of choice. In most yeast onychomycosis, which mainly affect the fingernails, amorolfine or cyclopiroxolamine lacquers are sufficient for healing. Onychomycosis caused by non-dermatophyte molds is infrequent, and to avoid confusion with occasional contaminants, diagnosis should always be confirmed with two or more positive cultures. Clinical cure is most likely combining oral and topical treatment.). When it is impossible to perform a systemic treatment or health problems arise during treatment and in some mycosis fungal opportunistic palliative procedures can be used as the removal of portions parasitized by the fungus by partial or total excision of the affected nails. Topical therapy should first be considered because of its few side effects. There are several antifungal preparations based on imidazoles, allylamines or polyenes. Chemical preparations with antifungal, antiseptic and often keratolytic properties have also been used, such as benzoic acid, benzoyl peroxide or salicylic acid, where there is evidence of its ineffectiveness. Creams, ointments or solutions do not diffuse well through the nail plate.

The products especially indicated for the nails are presented in the form of lacquers with which it is obtained that the antifúngico is in contact with the nail during a longer time. These doctors continue to say: "NATURAL" TREATMENTS" There is no "natural" method that has proven to be effective in the treatment of onychomycosis. Some of the supposed curative effects of homemade methods (vinegar washing, plant lotions, etc.) are due in part to the greater attention paid to nail care during these treatments (hygiene, elimination of hyperkeratotic zones, cutting Frequent, etc.). Ajoene, an organosulfur compound obtained from alcoholic extracts of garlic, has an antifungal effect, although there are not enough studies to support its usefulness. In a publication of Elsevier-magazine-pharmacy-professional -3-pdf-13095632-S300 November 2006, Drs. Joan Dalmau, Esther Roe and others, conclude: "In large nail hyperkeratosis many authors recommend, in addition to systemic treatment, the use of an antimytotic (for example, bifonazole) in combination with lacquered urea of nails to improve the penetration of the drug and to reduce the nail mass. A controversial issue is the extraction of the nail before starting the treatment. It is a painful measure that in the several studies analyzed does not provide greater efficiency in the treatment, reason why it is only possible to recommend a chemical extraction with urea to 40% and later topical treatment in those patients that for some reason cannot receive the systemic therapy. To date, there is no therapeutic agent that alone achieves 100% success. For this reason, the association of a systemic antifungal agent with another in nail polish for 3 and 6 months is the most effective treatment for onychomycosis, both in feet and hands.

### EXPLANATION OF THE INVENTION

It has been unexpectedly discovered that the stripped use of the plant leaf Sedum telephium together with a sulfanilamide powder solution has healing properties of very rapid effect against nail onychomycosis caused by fungi and yeasts.

The product obtained by the described process is used for direct topical application of the leaves or in the form of a cream or ointment on nails infected with onychomycosis.

In one embodiment, the invention is as explained below.

A leaf is separated from the plant Sedum telephium and the skin is removed from only one of its faces - preferably the beam. Once the blade has been stripped, a fine solution of sulfanilamide powder is applied to the surface and then applied to the infected nail by covering it completely, including a few millimeters around it. On the sheet is superimposed a gauze that completely occludes and on the gauze an adhesive strip or tape that firmly grasps the finger in his around and should remain so for at least 12 hours straight.

If the night is used it is more effective the treatment since the immobility is convenient to avoid the detachment of the plaster. Once this time is over, the sheet can be replaced if necessary. Depending on the progress of the infection, several days may be necessary, although experience shows that it is not necessary to apply the treatment beyond one week, at which time the infection completely disappears. If the patient needs to move, he can do so, but by the probable displacement of the plaster, healing can be delayed. Comparing the efficacy of this treatment with the current ones, their results are astonishing, since all studies show that onychomycosis is an infection that is usually very rebellious to current treatments, especially in the elderly or those with diabetes.

## Claims

1. A method of preparing a product based on the leaves of the Sedum telephium plant comprising the following steps:
a) Strip the skin that protects the leaf of the Sedum telephium plant from one side;
b) once stripped the sheet impregnated superficially with sulfanilamide powder.

2. A product based on the leaves of the Sedum telephium plant obtained according to the process steps of claim 1.

3. Use of the product according to claim 2 for manufacturing a topical medicament for the treatment of nails infected with onychomycosis.
